# EUROPEAN PATENT APPLICATION

(11) **EP 4 238 604 A1**
(43) Date of publication of application: **06.09.2023**
(21) Application number: 21884345.6
(22) Date of filing: 25.03.2021
(51) Int. Cl.: A61M 25/00, A61B 1/005

(54) **ADJUSTABLE BEND SHEATH**

(30) Priority: 30.10.2020 CN 202011199818; 30.10.2020 CN 202022470602 U
(71) Applicant: Hangzhou Valgen Medtech Co., Ltd., Hangzhou, Zhejiang 310052 (CN)
(72) Inventor: LI, Yang, Hangzhou, Zhejiang 310052 (CN); PENG, Bobo, Hangzhou, Zhejiang 310052 (CN); ZHANG, Tingchao, Hangzhou, Zhejiang 310052 (CN)
(74) Representative: Grassi, Stefano
(86) International application number: PCT/CN2021/083000
(87) International publication number: WO 2022/088601

(57) **Abstract**

The present application provides a bendable sheath, including a tube assembly and a handle assembly. The handle assembly includes an inner core, a fixing seat, and a driving member; the distal end of the tube assembly is provided with a bendable section, the proximal end of the tube assembly is fixedly connected to the inner core; the driving member is fixedly connected to the inner core; the driving member is rotatably connected to the fixing seat; one of the fixing seat and the driving member includes an elastic element, and the other includes an angle indexing part; the angle indexing part includes a plurality of indexing elements arranged at intervals; the elastic element moves among the plurality of indexing elements when the driving member rotates relative to the fixing seat. Because the proximal end of the tube assembly is fixedly connected to the inner core and the driving member is fixedly connected to the inner core, quantitative rotation of the driving member drives quantitative rotation of the tube assembly, so as to achieve precise regulation of the bending angle of the tube assembly, and prevent the occurrence of too much or insufficient rotation of the tube assembly.

## Description

The present application claims the priority of the Chinese patent application with the application number 202011199818.2 and 202022470602.7 filed on October 30, 2020, and the title of "Bendable Sheath", which were submitted to the China National Intellectual Property Administration on October 30, 2020, all the contents of the above-referenced applications are incorporated in the present application by reference.

### TECHNICAL FIELD

The present application relates to the technical field of medical devices, in particular to a bendable sheath.

### BACKGROUND

Transcatheter interventional therapy is a minimally invasive treatment, that is, under the guidance of medical imaging equipment, special catheters, guide wires and other precision instruments are introduced into the human body to diagnose and locally treat lesions in the body. In cardiac interventional therapy, the more representative ones are transcatheter mitral valve repair, transcatheter mitral valve replacement, transcatheter aortic replacement and so on. The aforementioned transcatheter interventional minimally invasive treatment operations all need to be assisted by bendable sheath to ensure that the operating instrument is delivered to the required position through the curved blood vessel, and then the minimally invasive operation is performed.

The prior art discloses a bendable guiding catheter, which includes a head end, a bend section, a deflectable section, a main body and a handle. The handle is provided with a first knob and a second knob, the first knob is used to control the rotation of the tube body of the catheter along the central axis of the main body, and the second knob is used to control the deflection of the deflectable section of the catheter. However, when the existing bendable catheter needs to adjust the direction of the catheter body itself, it can only directly rotate the body of the guiding catheter itself, and it is difficult to control the amount of rotation of the overall rotation of the guiding catheter, which is prone to excessive or insufficient rotation.

### SUMMARY

In view of this, the purpose of the present application is to overcome the deficiencies of the prior art and provide a bendable sheath capable of quantitatively regulating the bending angle of the tube body.

In order to solve the above technical problems, the present application provides an bendable sheath, including a tube assembly and a handle assembly, the handle assembly includes an inner core, a fixing seat and a driving member, the distal end of the tube assembly is provided with a bendable section, the proximal end of the tube assembly is fixedly connected to the inner core, the driving member is fixedly connected to the inner core, the driving member is rotatably connected to the fixing seat, one of the fixing seat and the driving member includes an elastic element, and the other includes an angle indexing part, the angle indexing part includes a plurality of indexing elements arranged at intervals, when the driving member rotates relative to the fixing seat, the elastic element moves among the plurality of indexing elements.

In the bendable sheath of the present application, one of the fixing seat and the driving member includes the elastic element, and the other includes the angle indexing part, when the driving member rotates relative to the fixing seat, the cooperation between the elastic element and the angle indexing part enables the driving member to rotate quantitatively. Since the proximal end of the tube assembly is fixedly connected to the inner core, and the driving member is fixedly connected to the inner core, the quantitative rotation of the driving member drives the quantitative rotation of the tube assembly to achieve precise regulation of the direction of the tube assembly's bending angle, preventing a situation that the overall rotation of the tube assembly is too much or not enough.

### BRIEF DESCRIPTION OF THE DRAWINGS

In order to describe the technical solutions in this embodiments of the present application more clearly, the following will briefly introduce the drawings needed in the implementation manners. Obviously, the drawings in the following description are some implementation manners of the present application, and for those skilled in the art, other drawings can also be obtained from these drawings without creative work.
Fig. 1 is a three-dimensional structure schematic illustration of a bendable sheath of the first embodiment of the present application;
Fig. 2 is a three-dimensional exploded schematic illustration of the bendable sheath in Fig. 1;
Fig. 3 is a three-dimensional exploded schematic illustration of the bendable sheath from another perspective in Fig. 1;
Fig. 4 is a three-dimensional exploded schematic illustration of the bendable sheath in Fig. 3 after the housing and the adjusting component are removed;
Fig. 5 is a three-dimensional structural schematic illustration of the tube assembly, inner core, sliding member and fixing member of the bendable sheath in Fig. 2;
Fig. 6 is a three-dimensional structure exploded schematic illustration of the adjusting component and the traction member in Fig. 2;
Fig. 7 is a sectional illustration of the tube assembly of the bendable sheath of the present application;
Fig. 8 is a three-dimensional assembly structure schematic illustration of the fixing seat and the driving member in Fig. 4;
Fig. 9 is a three-dimensional structure exploded schematic illustration of the fixing seat and the driving member in Fig. 8;
Fig. 10 is a three-dimensional structure exploded schematic illustration of the fixing seat from another perspective and the driving member in Fig. 9;
Fig. 11 is a sectional illustration of the fixing seat and the driving member in Fig. 8;
Fig. 12 is a further three-dimensional structure assembly schematic illustration of the bendable sheath of Fig. 2;
Fig. 13 is a front view of the bendable sheath in Fig. 1;
Fig. 14 is a sectional illustration along line XIV-XIV in Fig. 13;
Fig. 15 is a three-dimensional schematic illustration of the bendable sheath placed at the supporting accessories according to the first embodiment of the present application;
Fig. 16 is a three-dimensional exploded schematic illustration of the bendable sheath and the supporting accessories in Fig. 15;
Fig. 17 is a three-dimensional assembly structure schematic illustration of the fixing seat and the driving member of the bendable sheath according to the second embodiment of the present application;
Fig. 18 is a three-dimensional structure exploded schematic illustration of the fixing seat and the driving member in Fig. 17;
Fig. 19 is a three-dimensional structure exploded schematic illustration of the fixing seat from another perspective and the driving member in Fig. 18;
Fig. 20 is a sectional illustration of the fixing seat and the driving member in Fig. 17;
Fig. 21 is a three-dimensional assembly structure schematic illustration of the fixing seat and the driving member of the bendable sheath according to the third embodiment of the present application;
Fig. 22 is a three-dimensional structure exploded schematic illustration of the fixing seat and the driving member in Fig. 21;
Fig. 23 is a three-dimensional structure exploded schematic illustration of the fixing seat from another perspective and the driving member in Fig. 18;
Fig. 24 is a three-dimensional assembly structure schematic illustration of the fixing seat and the driving member of the bendable sheath according to the fourth embodiment of the present application;
Fig. 25 is a sectional illustration of the fixing seat and the driving member after assembly in Fig. 24;
Fig. 26 is a three-dimensional assembly structure schematic illustration of the fixing seat and the driving member of the bendable sheath according to the fifth embodiment of the present application.

### DETAILED DESCRIPTION

The following will clearly and completely describe the technical solutions in the embodiments of the present application with reference to the drawings in the embodiments of the present application. Apparently, the described embodiments are only some of the embodiments of the present application, not all of them. Based on the embodiments in the present application, all other embodiments obtained by ordinary technicians in the art without paying creative work fall within the scope of protection in the present application.

In the description of the present application, it should be noted that the orientations or positional relationships indicated by the terms "up", "down", "inside", "outside" and so on are based on the orientation or positional relationships shown in the drawings, which is only for the convenience of describing the present application and simplifying the description, rather than indicating or implying that the device or element referred to must have a specific orientation, be constructed and operated in a specific orientation, so it should not be construed as a limitation of the present application. In addition, the terms "first", "second" and the like are used for descriptive purposes only, and should not be construed as indicating or implying relative importance.

In order to describe the structure of the wire locking system and the wire locking device more clearly, the limited terms "proximal end", "distal end" and "axial direction" in the present application are commonly used terms in the field of interventional medicine. Specifically, "distal end" indicates one end away from an operator during a surgery; "distal end" indicates one end close to the operator during the surgery; the proximal end in the present application is closer to the operator (surgeon) than the distal end, after the device is assembled, each component therein includes a proximal end and a distal end, wherein the proximal end of each component is closer to the operator than the distal end. "Axial direction" refers to the direction of the central axis of the device, and the radial direction is the direction perpendicular to the central axis. Unless otherwise defined, all technical and scientific terms used in the present application have the same meaning as commonly understood by technical personnel in the technical field to which the present application belongs. The conventional terms used in the description of the present application are only for the purpose of describing specific embodiments, and should not be construed as limiting the present application.

It should be noted that when an element is referred to as being "fixed to" or "disposed at" another element, the element may be directly connected to the another element, or may be indirectly connected to the another element through one or more connecting elements. When an element is referred to as being "connected to" another element, it can be directly connected to the another element or connected to the another element through one or more connecting elements.

### First embodiment

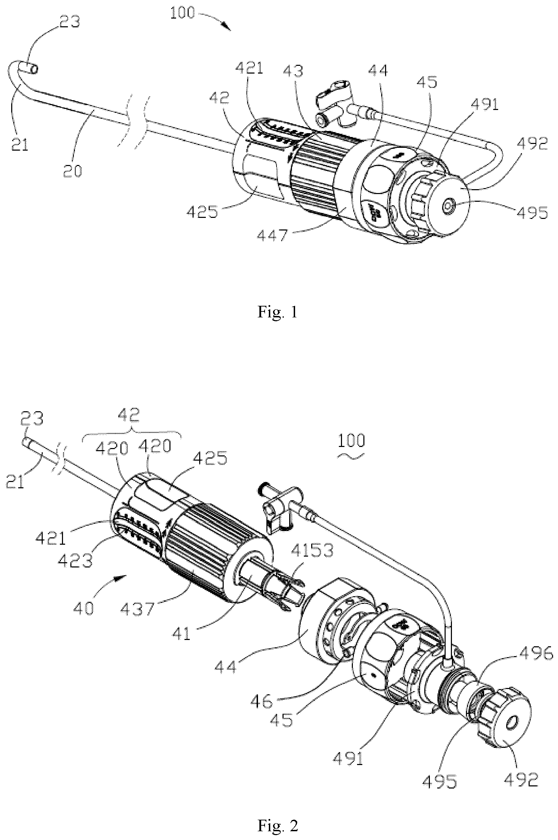

Please referring to Figs. 1 to 3, the present application provides a bendable sheath 100, including a tube assembly 20 and a handle assembly 40. The handle assembly 40 includes an inner core 41, a housing 42 sleeved at the inner core 41, a adjusting component 43 rotatably sleeved at the inner core 41, a fixing seat 44 and a driving member 45 disposed at the proximal end of the inner core 41. The distal end of the tube assembly 20 is provided with a bendable section 21, and the proximal end of the tube assembly 20 is fixedly connected to the inner core 41. The driving member 45 is fixedly connected to the inner core 41, and the driving member 45 is rotatably connected to the fixing seat 44. One of the fixing seat 44 and the driving member 45 includes an elastic element 46, and the other includes an angle indexing part 47. The angle indexing part 47 includes a plurality of indexing elements 471 arranged at intervals. When the driving member 45 rotates relative to the fixing seat 44, the elastic element 46 moves among the plurality of indexing elements 471.

In the bendable sheath 100 of the present application, one of the fixing seat 44 and the driving member 45 includes the elastic element 46, and the other includes the angle indexing part 47, when the driving member 45 rotates relative to the fixing seat 44, the cooperation between the elastic element 46 and the angle indexing part 47 enables the driving member 45 to rotate quantitatively. Since the proximal end of the tube assembly 20 is fixedly connected to the inner core 41, and the driving member 45 is fixedly connected to the inner core 41, the quantitative rotation of the driving member 45 drives the quantitative rotation of the tube assembly 20 to achieve precise regulation of the direction of the tube assembly's bending angle, preventing a situation that the overall rotation of the tube assembly 20 is too much or not enough.

It can be understood that the distal end of the tube assembly 20 is provided with the bendable section 21, and the adjusting component 43 is used to adjust the bending degree of the bendable section 21 so that the distal end of the tube assembly 20 forms the bending angle. When the driving member 45 rotates relative to the fixing seat 44, the elastic element 46 moves among the plurality of indexing elements 471, so that the driving member 45 drives the inner core 41 to rotate, and the inner core 41 rotates to drive the tube assembly 20 to rotate, so as to expediently adjust the direction of the bending angle of the bendable section 21. The proximal end of the tube assembly 20 and the inner core 41 can be connected as a whole by but not limited to bonding, locking or heat insulation, the tube assembly 20 and the inner core 41 are coaxial.

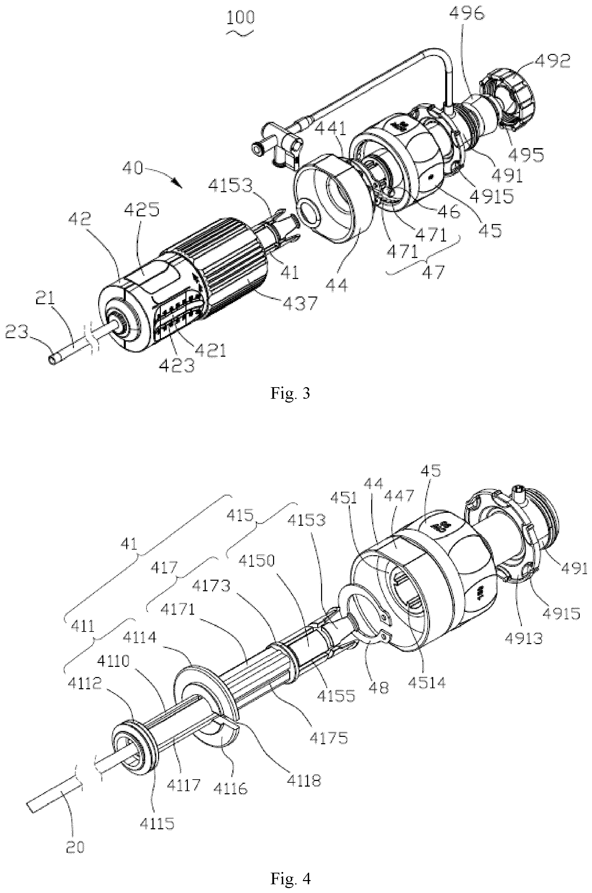

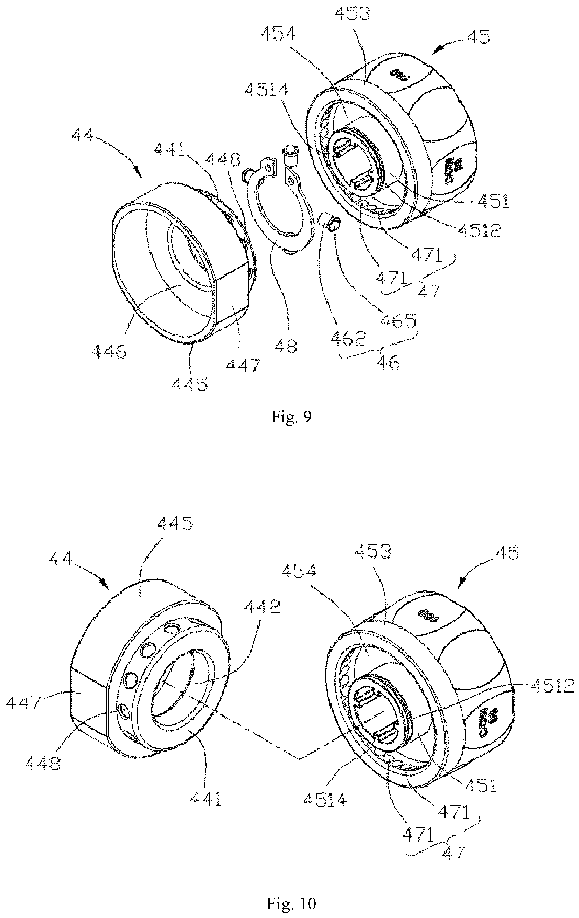

Please refer to Figs. 4 to 9 together, the tube assembly 20 includes an inner membrane 201, a reinforcing tube 202 sleeved at the inner membrane 201, and an outer tube 203 sleeved at the reinforcing tube 202. The inner membrane 201 can be a flexible tube made of flexible material such as polytetrafluoroethylene (PTFE) and so on, which is easy to bend. The reinforcing tube 202 can be a metal braided mesh structure or a metal cylinder cut mesh structure. The reinforcing tube 202 not only has a certain rigidity, but also can be bent in the axial direction, so as to provide support for the tube assembly 20 to prevent torsional deformation of the tube assembly 20 in the radial direction and do not affect the bending of the bendable section of the tube assembly 20. The outer tube 203 is made of a material with a certain hardness such as block polyether amide resin (PEBAX) to protect the tube assembly 20. The hardness of the outer tube 203 corresponding to the bendable section 21 is smaller than that of other parts of the outer tube 203, so as to protect the tube assembly 20 while preventing impact on the bending of the bendable section 21. The inner membrane 201, the reinforcing tube 202 and the outer tube 203 are formed together by hot melt forming to form at least one delivery lumen 205 completely penetrating from the proximal end to the distal end along the axis of the tube assembly 20. The end of the tube assembly 20 is provided with a guiding head 23 with a smooth surface, that is, a Tip, and a marker ring (not shown in the figure) is disposed adjacent to the end of the Tip. The marker ring includes but not limited to a tantalum ring and so on, so that it can accurately know whether the distal end of the tube assembly 20 has reached the designated position under the medical image equipment.

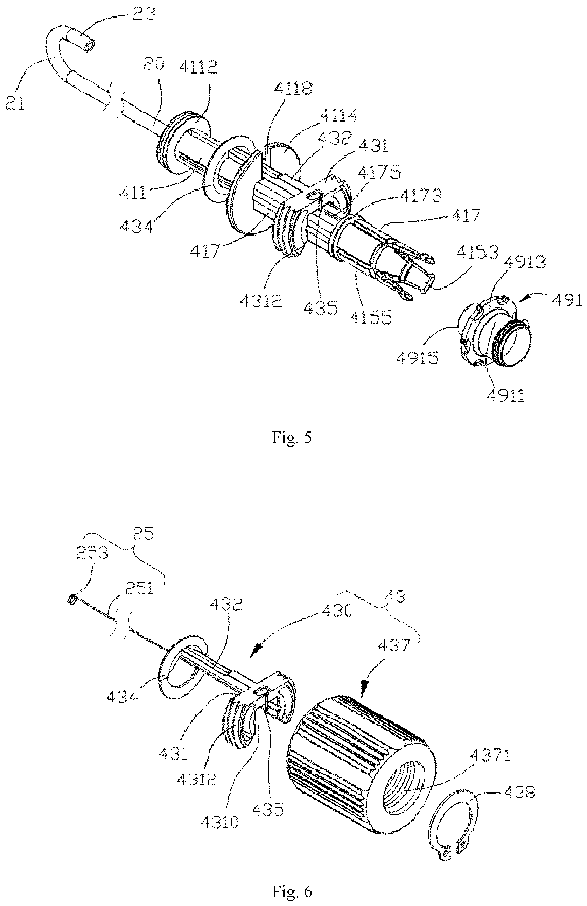

As shown in Fig. 5 and Fig. 6, the tube assembly 20 further includes a traction member 25 connected to the bendable section 21. The traction member 25 includes a traction wire 251 and an anchor ring 253 disposed at the distal end of the traction wire 251. The anchor ring 253 is connected to the bendable section 21, preferably, the anchor ring 253 is coaxial with the bendable section 21. Specifically, the anchor ring 253 can be welded with the bendable section 21 as a whole. Ways to connected the distal end of the traction wire 251 to the anchor ring 253 include but are not limited to bonding, welding, thermal fusion or knotting. The tube wall of the tube assembly 20 defines a lumen in the axial direction, and the traction wire 251 is movably accommodated in the lumen of the tube assembly 20. Specifically, the lumen can be disposed in the tube wall of the reinforcing tube 202 or the outer tube 203 of the tube assembly 20, that is, the lumen is embedded in the tube wall of the reinforcing tube 202 or the outer tube 203. The traction wire 251 is used to pull the bendable section 21 to bend or release the pulling force on the traction wire 251 to restore the bendable section 21 to straightness. The traction wire 251 can be made of metal materials, such as stainless steel, tungsten alloy, cobalt-chromium alloy or nickel-titanium alloy and so on, or can be made of polymer material with certain strength. The proximal end of the traction wire 251 can be connected to the adjusting component 43, so that the traction wire 251 can pull the bendable section 21 to bend or restore straightness by operating the adjusting component 43 .

As shown in Fig. 4 and Fig. 5, the inner core 41 includes a positioning part 411 at the distal end, a connecting part 415 at the proximal end, and a guiding part 417 between the positioning part 411 and the connecting part 415. Specifically, the positioning part 411 includes a distal core tube 4110, a first positioning ring 4112 fixedly sleeved at the distal end of the distal core tube 4110, and a second positioning ring 4114 fixedly sleeved at the proximal end of the distal core tube 4110. The outer peripheral wall of the first positioning ring 4112 is provided with at least one snapping groove 4115 along the circumferential direction, and one side of the second positioning ring 4114 facing the first positioning ring 4112 is provided with at least one positioning groove 4116 along the circumferential direction. The outer peripheral wall of the distal core tube 4110 is provided with a through groove 4117 along the axial direction, and the second positioning ring 4114 is provided with a gap 4118 communicating the through groove 4117 along its radial direction. The guiding part 417 includes a guiding tube 4171 fixedly connected to the proximal end of the distal core tube 4110, and a stop piece 4173 disposed at the proximal end of the guiding tube 4171. The lumen of the guiding tube 4171 is communicated to the lumen of the distal core tube 4110. The outer peripheral wall of the guiding tube 4171 is provided with a guiding groove 4175 communicating the through groove 4117 along the axial direction. The connecting part 415 includes a proximal core tube 4150 connected to one side of the stop piece 4173 away from the guiding tube 4171 and a plurality of locking pieces 4153 disposed at the proximal end of the proximal core tube 4150. The lumen of the proximal core tube 4150 is axially communicated to the lumen of the slide guide tube 4170. The plurality of locking pieces 4153 are arranged in a circle along the circumferential direction of the proximal core tube 4150. The proximal end of the inner core 41 is further provided with a plurality of external locking slots 4155, specifically, the outer peripheral wall of the proximal core tube 4150 is provided with the plurality of locking slots 4155 along the axial direction, and the plurality of locking slots 4155 are arranged in a circle along the circumferential direction of the proximal core tube 4150.

As shown in Figs. 2 to 6, the adjusting component 43 includes a sliding member 430 and a adjusting member 437 sleeved at the sliding member 430. The proximal end of the traction wire 251 is fixedly connected to the sliding member 430, and the sliding member 430 is slidably sleeved at the guiding part 417 of the inner core 41 along the axial direction. The sliding member 430 and the adjusting member 437 are screwed together, and the sliding member 430 is driven to move along the axial direction of the inner core 41 by rotating the adjusting member 437, so as to drive the traction wire 251 to pull the bendable section 21 to bend or restore to straightness .

Specifically, the sliding member 430 includes a sliding block 431 sleeved at the guiding part 417, a connecting rod 432 disposed at the distal end of the sliding block 431, an identification member 434 disposed at the distal end of the connecting rod 432, and a fixing block 435 connected to the sliding block 431. The sliding block 431 is provided with a notch 4310 along the axial direction, and the guiding part 41 is accommodated in the notch 4310. The fixing block 435 is disposed at the inner wall of the notch 4310 and extends radially to the notch 4310, and the fixing block 435 is used for fixing and connecting the proximal end of the traction wire 251. It can be understood that the end of the fixing block 435 is provided with a fixing aperture along the axial direction, and the proximal end of the traction wire 251 is fixedly connected in the fixing aperture. The fixing block 435 and the sliding block 431 can be fixedly connected by locking, screwing or glueing, or can be integrally formed. The sliding block 431 is provided with an external thread 4312, and the adjusting member 437 is provided with an inner cavity that penetrates through its proximal end surface and the distal end surface in the axial direction, and the inner peripheral wall of the inner cavity of the adjusting member 437 is provided with an internal thread 4371 that cooperates with the external thread 4312. The identification member 434 can be composed of two detachable semi-circular rings, one of which is fixedly connected to the distal end of the connecting rod 432 of the sliding block 431 for easy installation.

The housing 42 is composed of two frame bodies 420 which are detachably integrated, at least one of the outer peripheral walls of the frame bodies 420 is provided with an observation window 421 along the axial direction., and the one of the frame bodies 420 is provided with an indication scale 423 on at least one side of the observation window 421, and the indication scale 423 is used to indicate the position of the identification member 434 to measure the length of the sliding member 430 sliding along the axial direction. Specifically, confirming the scale of the identification member 434 corresponding to the position of the indication scale 423 by the observation window 421, the bending degree corresponding to the bendable section 21 can be known through the reading of the scale. A positioning recess 425 is formed at the intersection of the outer peripheral walls of the two frame bodies 420 .

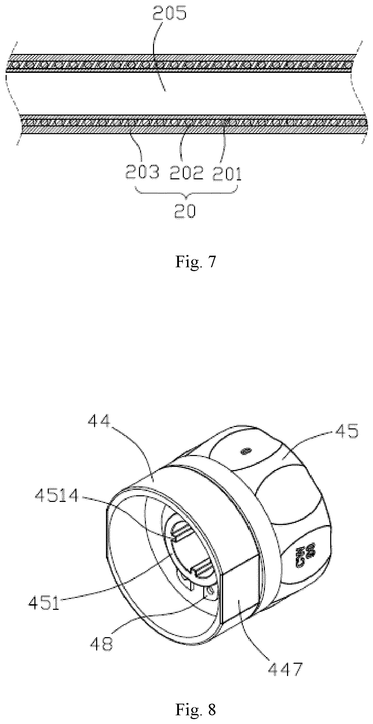

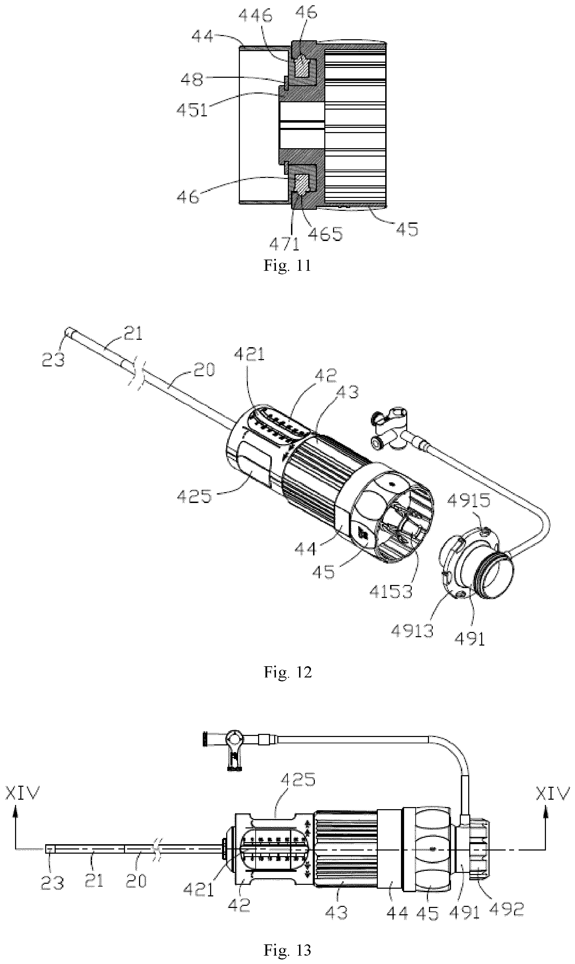

Please refer to Figs. 8 to 11 together, the fixing seat 44 includes a connecting ring 441, and the connecting ring 441 includes a connecting aperture 442 penetrating through the fixing seat 44 in the axial direction. The driving member 45 includes a connecting cylinder 451, and the connecting cylinder 451 is rotatably disposed in the connecting aperture 442. In this way, the driving member 45 is rotatably connected to the fixing seat 44. In this embodiment, the fixing seat 44 further includes a fixing ring 445 disposed at one end of the connecting ring 441. The fixing ring 445 is coaxial with the connecting ring 441, the inner cavity of the fixing ring 445 is communicated to the inner cavity of the connecting ring 441, the inner diameter of the fixing ring 445 is greater than the inner diameter of the connecting ring 441, and the outer diameter of the fixing ring 445 is greater than the outer diameter of the connecting ring 441, so that the fixing seat 44 form a circular stepped structure, that is, a step 446 is formed at the inner cavity of the fixing ring 445 close to the connecting ring 441. The outer peripheral wall of the fixing ring 445 is provided with two symmetrical fixing surfaces 447, and each fixing surface 447 extends along the axial direction of the fixing ring 445. The outer peripheral wall of the connecting ring 441 defines at least two spaced installation apertures 448, and each installation aperture 448 extends along the radial direction of the connecting ring 441. Preferably, the outer peripheral wall of the connecting ring 441 is provided with at least two symmetrical installation apertures 448. The elastic element 46 is disposed at the outer peripheral wall of the connecting ring 441, and in this embodiment, the elastic element 46 is installed in the installation aperture 448. Specifically, the elastic element 46 can be a spring plunger, and the installation aperture 448 is used for installing the spring plunger. The spring plunger includes a housing 462, a spring and a steel ball 465 located in the housing 462. the spring is connected to the steel ball 465, and the elasticity of the spring can be used to push the steel ball 465 to move.

The driving member 45 further includes a driving cylinder 453 coaxial with the connecting cylinder 451, and the connecting cylinder 451 is accommodated in the driving cylinder 453. The end of the connecting cylinder 451 and the driving cylinder 453 are connected together through a connecting board. An annular groove 454 is formed between the driving cylinder 453 and the connecting cylinder 451, and the connecting ring 441 is rotatably accommodated in the annular groove 454. Specifically, the end of the outer peripheral wall of the connecting cylinder 451 away from the connecting board is provided with a snapping groove 4512 along the circumferential direction. The inner peripheral wall of the connecting cylinder 451 is provided with a plurality of locking ribs 4514 along the axial direction, and the plurality of locking ribs 4514 are arranged along the circumferential direction of the connecting cylinder 451. The plurality of indexing elements 471 are arranged at the inner peripheral wall of the driving cylinder 453, and the plurality of indexing elements 471 can be arranged at equal or unequal intervals. In this embodiment, the plurality of indexing elements 471 are a plurality of concave grooves arranged at intervals at the inner peripheral wall of the driving cylinder 453. When the driving member 45 rotates relative to the fixing seat 44, the elastic element 46, that is, the spring plunger, is successively snapped into the plurality of concave grooves. The plurality of concave grooves may be evenly arranged in a circle along the circumferential direction of the driving cylinder 453, that is, the plurality of concave grooves are arranged at equal intervals along the circumferential direction of the driving cylinder 453. Each concave groove can be matched with the elastic element 46, in this embodiment, each concave groove is matched with the steel ball 465, that is, the steel ball 465 can be snapped into the concave groove or disengaged from the concave groove. If the number of indexing elements 471 disposed at the inner peripheral wall of the driving cylinder 453 is N, the degree between every two adjacent indexing elements 471 is 360 divided by N. The outer peripheral wall of the driving cylinder 453 is provided with degree markings along its circumferential direction. In this embodiment, the outer peripheral wall of the driving cylinder 453 is marked with 0 degrees, 90 degrees, 180 degrees, 270 degrees and so on, for the user to judge the rotation angle of the driving member 45 relative to the fixing seat 44.

When assembling the fixing seat 44 and the driving member 45, the elastic element 46 is first snapped into the corresponding installation aperture 448. Since the elastic element 46 is in interference fit with the installation aperture 448, it can be ensured that the elastic element 46 will not disengage from the installation aperture 448. Secondly, the connecting ring 441 of the fixing seat 44 is rotatably accommodated in the annular groove 454, and the connecting cylinder 451 passes through the connecting aperture 442 and exposes the snapping groove 4512. Specifically, the end of the connecting cylinder 451 is accommodated in the inner cavity of the fixing ring 445, and snapping groove 4512 is exposed from the step 446. Then, the circlip 48 of the handle assembly 40 is snapped into the snapping groove 4512, so that the fixing seat 44 and the driving member 45 are locked and connected. At this time, the fixing seat 44 and the driving member 45 can rotate relative to each other, that is, when the fixing seat 44 is fixed, the driving member 45 can rotate in both directions relative to the fixing seat 44, but the fixing seat 44 and the driving member 45 cannot shift relative to each other in the axial direction, that is, the fixing seat 44 and the driving member 45 cannot shift independently in the axial direction. When the driving member 45 rotates relative to the fixing seat 44, the steel balls 465 of the elastic elements 46 are sequentially snapped into the plurality of concave grooves; When the driving member 45 does not rotate relative to the fixing seat 44, the steel balls 465 of the elastic elements 46 are embedded into the corresponding concave grooves to achieve self-locking, that is, to maintain the relative positioning of the fixing seat 44 and the driving member 45 without external force, so as to always protect the bending direction of the bendable section 21 of the tube assembly 20.

In other embodiments, the elastic element 46 can be disposed at the inner peripheral wall of the driving cylinder 453, and the plurality of indexing elements 471 can be disposed at the outer peripheral wall of the connecting ring 441. Specifically, the inner peripheral wall of the driving cylinder 453 is provided with at least two spaced installation apertures, each installation aperture is provided with the elastic element 46, and the elastic element 46 is the spring plunger. The outer peripheral wall of the connecting ring 441 is provided with the plurality of indexing elements 471 along its circumferential direction, and the plurality of indexing elements 471 are the plurality of concave grooves arranged at intervals.

In other embodiments, the elastic element 46 is an elastic protrusion, and the elastic protrusion can be disposed at the outer peripheral wall of the connecting ring 441 or the inner peripheral wall of the driving cylinder 453. The plurality of indexing elements 471 are the plurality of concave grooves. Specifically, when two or more spaced elastic protrusions are disposed at the outer peripheral wall of the connecting ring 441, the plurality of concave grooves are disposed at the inner peripheral wall of the driving cylinder 453 and arranged in a circle along the circumferential direction of the driving cylinder 453. When two or more spaced elastic protrusions are disposed at the inner peripheral wall of the driving cylinder 453, the plurality of concave grooves are disposed at the outer peripheral wall of the connecting ring 441 and arranged in a circle along the circumferential direction of the connecting ring 441. When the driving member 45 rotates relative to the fixing seat 44, the elastic protrusions snap into the plurality of concave grooves sequentially. When the driving member 45 does not rotate relative to the fixing seat 44, the elastic protrusions are embedded into the corresponding concave grooves, so as to achieve self-locking.

Please refer to Figs. 2 to 5 together, the handle assembly 40 further includes a fixing member 491, a sealing nut 492, a silicone plug 494 and a sealing support 496. The fixing member 491 is provided with a locking interface 4915, the proximal end of the inner core 41 passes through the connecting cylinder 451 and exposes the plurality of locking pieces 4153, and the plurality of locking pieces 4153 are inserted into the locking interface 4915. Specifically, the fixing member 491 includes a fixing cylinder 4911 and an annular snapping sheet 4913 fixedly sleeved at the fixing cylinder 4911, one end of the outer peripheral wall of the fixing cylinder 4911 is provided with an external thread, and the end of the fixing cylinder 4911 away from the external thread is provided with a locking interface 4915. The sealing nut 492 is provided with an internal thread matched with the fixing cylinder 4911. The silicone plug 494 is installed at the fixing member 491 through the sealing support 496. The lumen of the bendable sheath 100 is used to accommodate and pass through the delivery sheath of the interventional medical device, the sealing nut 492 and the silicone plug 494 can be wrapped around the delivery sheath to seal the delivery sheath, so as to ensure that no blood leakage during the operation.

Please refer to Figs. 1 to 6 and Figs. 12 to 14 together, when assembling the bendable sheath 100, firstly, install the sliding member 430 at the inner core 41. Specifically, the sliding block 431 is sleeved at the guiding part 417, and the fixing block 435 is accommodated in the guiding groove 4175, the proximal end of the traction wire 251 is fixedly connected to the fixing block 435, and the connecting rod 432 is passed through in the gap 4118 of the second positioning ring 4114, the identification member 434 is buckled at the positioning part 411. The sliding block 431 can slide along the axial direction of the inner core 41 to drive the identification member 434 to slide axially, and the sliding block 431 is stopped between the second positioning ring 4114 and the stop piece 4173. Secondly, two frame bodies 420 are buckled at the positioning part 411 of the inner core 41, so that the distal wall of each frame body 420 is snapped into the snapping groove 4115 of the first positioning ring 4112, and the proximal end of each frame body 420 is snapped into the positioning groove 4116 of the second positioning ring 4114. Thirdly, the adjusting member 437 is threaded with the sliding block 431 to be disposed at the inner core 41, and the circlip 4370 is locked to the proximal end of the stop piece 4173 of the inner core 41, so that the adjusting member 437 stops between the second positioning ring 4114 and the circlip, the axial shift of the adjusting member 437 is limited, that is, the adjusting member 437 can only rotate around the axis of the inner core 41. Then, the fixing seat 44 and the driving member 45 installed integrally are installed to the proximal end of the inner core 41, so that the proximal end of the inner core 41 passes through and is fixedly connected to the connecting cylinder 451. Specifically, the connecting part 415 is inserted into the inner cavity of the connecting cylinder 451 through the inner cavity of the fixing seat 44, and the plurality of locking ribs 4514 are respectively locked into the plurality of locking slots 4155 of the proximal core tube 4150, and the plurality of locking ribs 4514 and the plurality of locking slots 4155 are corresponding to each other and connected with each other. Finally, install the fixing member 491 to the proximal end of the inner core 41, specifically, insert the plurality of locking pieces 4153 at the proximal end of the inner core 41 into the locking interface 4915 of the fixing cylinder 4911 and lock them to the inner peripheral wall of the fixing cylinder 4911, to lock the driving member 45 and the inner core 41; the silicone plug 495 is installed at the proximal end of the fixing member 491 through the sealing support 496, and the sealing nut 492 is threaded with the fixing member 491.

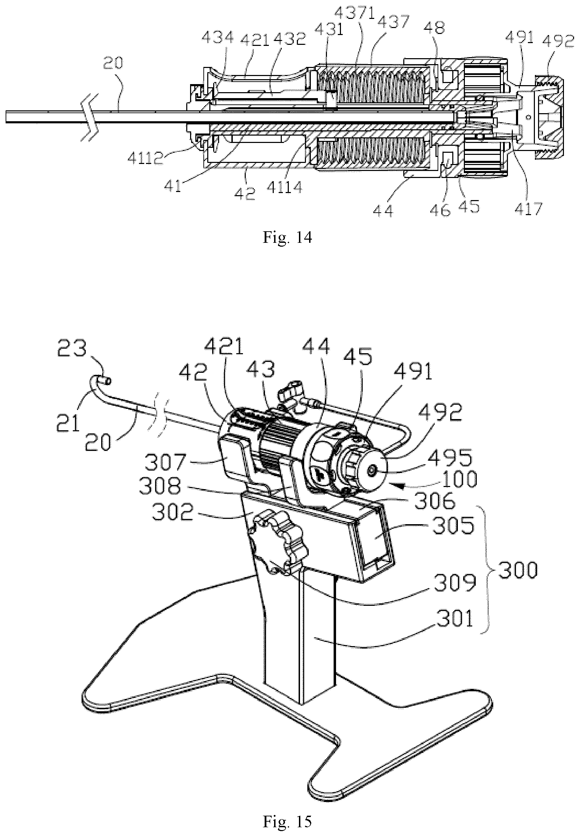

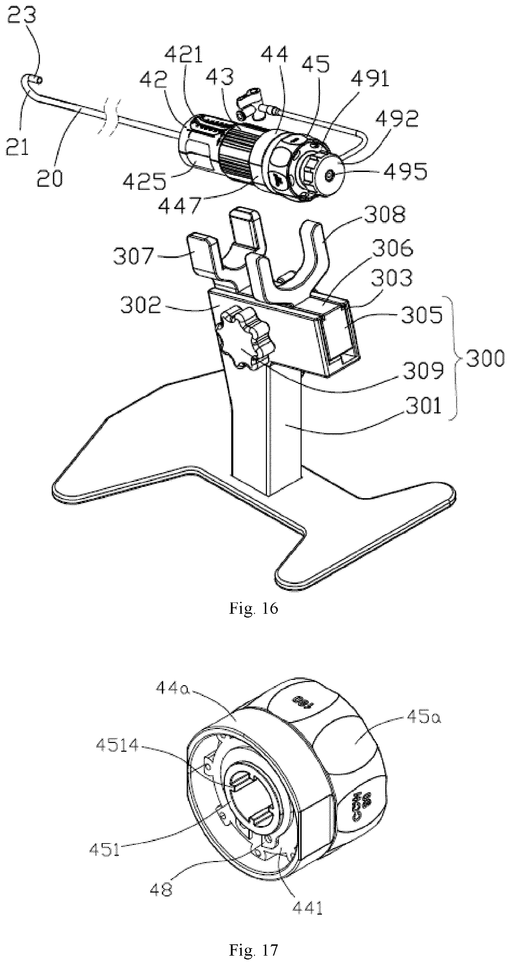

As shown in Fig. 15 and Fig. 16, the adjusting component 43 is used to adjust the bending degree of the bendable section 21 of the tube assembly 20, so that the distal end of the tube assembly 20 forms a bending angle, and the cooperation of the fixing seat 44 and the driving member 45 is used to adjust the direction of the bending angle of the bendable section 21. When using the bendable sheath 100, the housing 42 and the fixing seat 44 need to be fixed by the supporting accessories 300 or the operator holding. The supporting accessories 300 includes a support frame 301, a bracket 305 slidably disposed at the support frame 301, and a locking member 309 for positioning the bracket 305 at the support frame 301. The support frame 301 includes a support bar 302 extending obliquely, and the support bar 302 has a guiding groove 303 along its length direction. The bracket 305 includes a sliding bar 306 slidably accommodated in the guiding groove 303, a first positioning fork 307 and a second positioning fork 308 arranged at intervals at the sliding bar 306, the first positioning fork 307 and the second positioning fork 308 are used to fix the housing 42 and the fixing seat 44 respectively. The locking member 309 passes through the supporting bar 302 and abuts against the sliding bar 306 in the guiding groove 303 to position the bracket 305. The housing 42 and the fixing seat 44 are fixed at the bracket 305, specifically, the first positioning fork 307 is locked with the positioning recess 425 of the housing 42, and the second positioning fork 308 is locked with the fixing surface 447 of the fixing seat 44.

When performing interventional surgery, for the convenience of operation, the bracket 305 is used to fix the fixing seat 44 and the housing 42, so that the interface of the indication scale 423 of the housing 42 is always upward. When the tube assembly 20 enters the patient's body, if it is necessary to adjust the direction of the bending angle of the bendable section 21, the inner core 41 can be driven to rotate by rotating the driving member 45, and the rotating direction of the driving member 45 can be clockwise or counterclockwise, and it can be rotated according to the actual needs, so as to adjust the direction of the bending angle of the bendable section 21.

During the process of rotating the driving member 45, the elastic element 46 moves among the plurality of indexing elements 471 to adjust the rotation degree of the inner core 41, thereby adjusting the direction of the bending angle of the bendable section 21. By arranging the plurality of indexing elements 471 at equal intervals at the inner peripheral wall of the driving member 45, the direction of the tube assembly 20 can be controlled equally and indexed, and at the same time, the magnitude of the rotational force can be controlled by the elastic deformation of the elastic element 46. Therefore, in the process of using the bendable sheath 100, it is not necessary to rotate the entire handle assembly 40, but the adjusting member 437 and/or the driving member 45 can be rotated independently, so that the housing 42 and the fixing seat 44 are static in place, which is convenient to use and operate, and the amount of overall rotation of the tube assembly 20 can be accurately controlled to avoid excessive or insufficient rotation, improve the success rate of the operation, and reduce the risk of the operation.

Of course, when using the bendable sheath 100, the supporting accessories 300 is not necessary, and the operator can directly hold the housing 42 and/or the fixing seat 44.

### Second embodiment

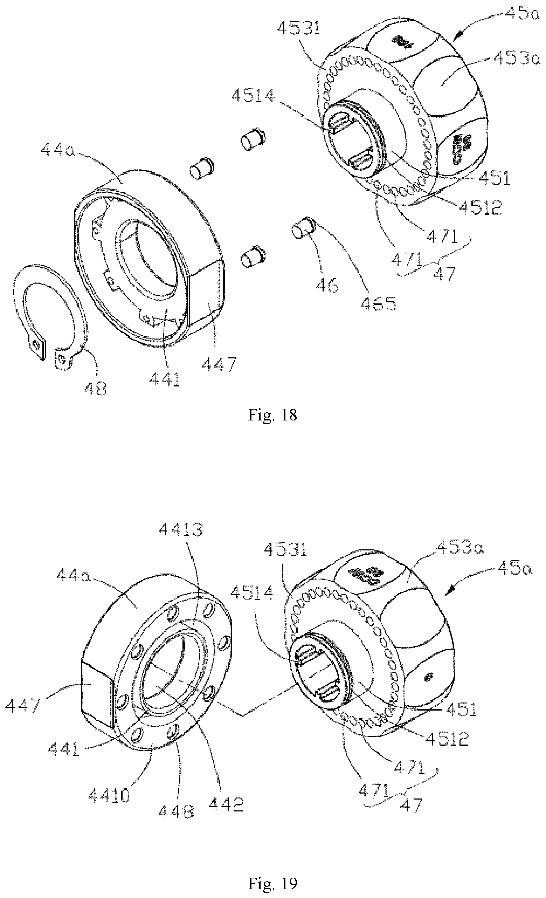

Please refer to Figs. 17 to 20 together, the structure of the second embodiment of the bendable sheath provided in the present application is similar to that of the first embodiment, the difference is that: the structure of the fixing seat 44a and the driving member 45a in the second embodiment is slightly different from the structure of the fixing seat 44 and the driving member 45 in the first embodiment, as follows:

In the second embodiment, the connecting ring 441 includes a first side wall 4410 facing the driving member 45a, and the connecting aperture 442 extending through the fixing seat 44a is formed in the middle of the first side wall 4410 in the axial direction. The driving member 45a includes a driving part 453a connected to the connecting cylinder 451, the driving part 453a is provided with a second side wall 4531 facing the first side wall 4410 around the connecting cylinder 451. The elastic element 46 is disposed at the first side wall 4410, the plurality of indexing elements 471 are disposed at the second side wall 4531. Specifically, the fixing seat 44a is a circular cylinder, and the first side wall 4410 of the fixing seat 44a is provided with at least two spaced installation apertures 448, and each installation aperture 448 extends along the axial direction of the connecting ring 441. Preferably, the first side wall 4410 is provided with at least two symmetrical installation apertures 448. In this embodiment, the elastic element 46 is installed in the installation aperture 448, specifically, the elastic element 46 is the spring plunger. The installation aperture 448 is used for installing the spring plunger. The first side wall 4410 of the fixing seat 44a is further provided with an annular groove 4413 to keep space and reduce the wall thickness, and facilitate the die sinking. Certainly, the annular groove 4413 may not be provided in the fixing seat 44a. The connecting cylinder 451 protrudes from the middle of the second side wall 4531 of the driving part 453a, and the inner cavity of the connecting cylinder 451 is communicated to the inner cavity of the driving part 453a. The plurality of indexing elements 471 are disposed at the second side wall 4531 along the circumferential direction of the connecting cylinder 451, and the plurality of indexing elements 471 can be arranged at equal or unequal intervals. In this embodiment, the plurality of indexing elements 471 are the plurality of concave grooves arranged at intervals at the second side wall 4531, when the driving member 45a rotates relative to the fixing seat 44a, the elastic element 46, namely the spring plunger, is snapped into the plurality of concave grooves sequentially. The plurality of concave grooves may be evenly arranged in a circle along the circumferential direction of the connecting cylinder 451, that is, the plurality of concave grooves are arranged at equal intervals along the circumferential direction of the connecting cylinder 451. Each concave groove can be matched with the elastic element 46, in this embodiment, each concave groove is matched with the steel ball 465 of the elastic element 46, that is, the steel ball 465 can be embedded into the concave groove or disengaged from the concave groove. The method of application and beneficial effects of the second embodiment are the same as those of the first embodiment, and will not be repeated here.

In other embodiments, the elastic element 46 is disposed at the second side wall 4531 of the driving cylinder 453a, and the plurality of indexing elements 471 are disposed at the first side wall 4410 of the connecting ring 441. Specifically, at least two spaced installation apertures are provided at the second side wall 4531 along the circumferential direction of the connecting cylinder 451, each installation aperture is provided with the elastic element 46, and the elastic element 46 is the spring plunger. The first side wall 4410 is provided with the plurality of indexing elements along its circumferential direction, and the plurality of indexing elements are the plurality of concave grooves arranged at intervals.

In other embodiments, the elastic element 46 is the elastic protrusion, and the elastic protrusion can be disposed at the first side wall 4410 of the connecting ring 441 or the second side wall 4531 of the driving cylinder 453a. The plurality of indexing elements 471 are the plurality of concave grooves. Specifically, when two or more spaced elastic protrusions are disposed at the first side wall 4410, the plurality of concave grooves are disposed at the second side wall 4531 and arranged in a circle along the circumferential direction of the connecting cylinder 451. When two or more spaced elastic protrusions are disposed at the second side wall 4531, the plurality of concave grooves are disposed at the first side wall 4410 and arranged in a circle along the circumferential direction of the connecting ring 441. When the driving member 45a rotates relative to the fixing seat 44a, the elastic protrusions are sequentially snapped into the plurality of concave grooves. When the driving member 45a does not rotate relative to the fixing seat 44a, the elastic protrusions are embedded into the corresponding concave grooves, so as to achieve positional self-locking.

### Third embodiment

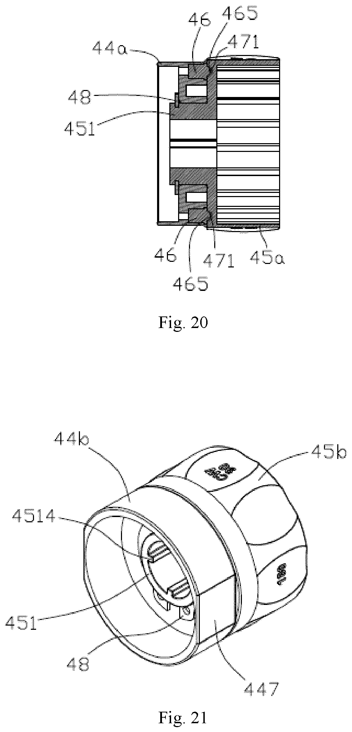

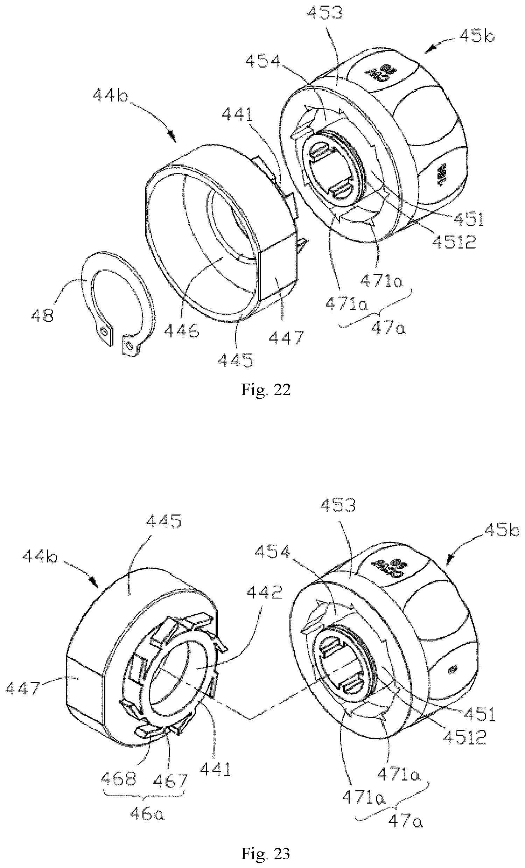

Please refer to Figs. 21 to 23 together, the structure of the third embodiment of the bendable sheath provided in the present application is similar to that of the first embodiment, the difference is that: the structure of the fixing seat 44b and the driving member 45b in the third embodiment is slightly different from the structure of the fixing seat 44 and the driving member 45 in the first embodiment, as follows:

In the third embodiment, the elastic element 46a is a convex rib with elasticity, and the plurality of indexing elements 471a of the angle indexing part 47a are a plurality of grooves arranged at intervals. When the driving member 45b rotates relative to the fixing seat 44b, the elastic convex ribs are sequentially snapped into the plurality of grooves. When the driving member 45b does not rotate relative to the fixing seat 44b, the elastic convex ribs are embedded into the corresponding grooves. Specifically, the outer peripheral wall of the connecting ring 441 of the fixing seat 44b is provided with a plurality of deformable elastic elements 46a along the circumferential direction of the connecting ring 441. The elastic element 46a is an inclined convex rib, the convex rib is flexible, and can elastically deform when squeezed. Each elastic element 46a includes a connecting block 467 protruding from the outer peripheral wall of the connecting ring 441 and an elastic positioning strip 468 obliquely extending from the end of the connecting block 467 to one side. The inclination angle of the elastic positioning strip 468 relative to the corresponding connecting block 467 is within a range of 0° to 90° . Preferably, the inclination angle of the elastic positioning strip 468 relative to the corresponding connecting block 467 is about 30 degrees. In this embodiment, the plurality of elastic elements 46a are arranged in a circle at even intervals along the circumferential direction of the connecting ring 441 at the outer peripheral wall of the connecting ring 441, that is, a plurality of elastic positioning strips 468 are arranged in a circle at even intervals along the circumferential direction of the connecting ring 441. An annular groove 454 is formed between the driving cylinder 453 and the connecting cylinder 451, and the indexing element 471a is a groove disposed at the inner peripheral wall of the driving cylinder 453 and faced the annular groove 454. In this embodiment, the inner peripheral wall of the driving cylinder 453 is provided with the plurality of grooves arranged in a circle along the circumferential direction of the driving cylinder 453, and the plurality of grooves can be matched with the convex ribs.

When assembling the fixing seat 44b and the driving member 45b, primarily, the connecting cylinder 451 of the driving member 45b is rotatably accommodated in the connecting aperture 442 of the fixing seat 44b and exposed outside the snapping groove 4512, the connecting ring 441 is accommodated in the annular groove 454 at the same time, and the convex ribs of the fixing seat 44b match the grooves of the driving member 45b. Then, the circlip 48 of the handle assembly 40 is snapped into the snapping groove 4512, so that the fixing seat 44b and the driving member 45b are locked and connected. At this time, the driving member 45b can rotate in one direction relative to the fixing seat 44b, but the fixing seat 44b and the driving member 45b cannot shift relative to each other in the axial direction. The installation method of the fixing seat 44b, the driving member 45b and the inner core 41 is the same as that of the first embodiment, and will not be repeated here.

The method of application and beneficial effects of the third embodiment of the bendable sheath are similar to those of the first embodiment, the difference is that: when the fixing seat 44b is fixed by the bracket 305 or the operator holds it, the driving member 45b rotates in one direction relative to the fixing seat 44b, that is, the driving member 45b rotates along the direction of the inclination angle of the convex rib, and the elastic positioning strips 468 are snapped into corresponding the plurality of grooves in turn. When the driving member 45b is not required to be rotated, the elastic positioning strip 468 is embedded into the groove to realize self-locking. For example, when rotating to a certain position, the plurality of elastic positioning strips 468 are snapped into the plurality of grooves respectively to realize the self-locking function, which is beneficial to the surgery.

When doing interventional surgery, the bracket 305 can be used to fix the fixing seat 44b. When the tube assembly 20 enters the patient's body, if it is necessary to adjust the direction of the bending angle of the bendable section 21, the inner core 41 can be driven to rotate along the inclined direction of the convex rib of the fixing seat 44b by rotating the driving member 45b, thereby adjusting the direction of the bending angle of the bendable section 21. Due to the evenly distributed convex rib structure at the fixing seat 44b, it is possible to achieve accurate and equal control of the direction of the bending angle of the tube assembly 20 .

In other embodiments, the number of convex ribs at the outer peripheral wall of the connecting ring 441 may be two or more, the number of grooves at the inner peripheral wall of the driving cylinder 453 is multiple, and the plurality of grooves are arranged with uniform or non-uniform spacing along the circumferential direction of the driving cylinder 453.

In other embodiments, the angle indexing part may be a toothed ring, and the toothed ring is arranged around the inner peripheral wall of the driving cylinder 453 in a circle. The toothed ring includes a plurality of toothed grooves as the indexing elements, and the plurality of toothed grooves can be matched with the convex rib.

### Fourth embodiment

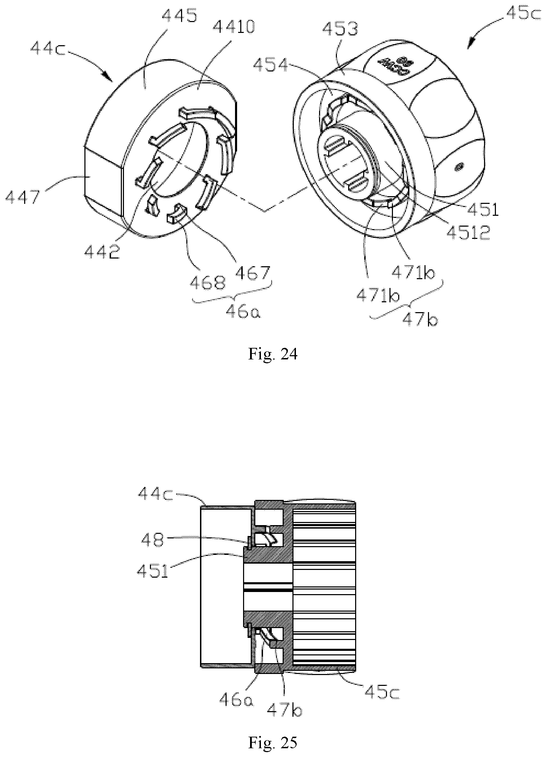

Please refer to Fig. 24 and Fig. 25 together, the structure of the fourth embodiment of the bendable sheath provided in the present application is similar to that of the third embodiment, the difference is that: the structure of the fixing seat 44c and the driving member 45c in the fourth embodiment is slightly different from the structure of the fixing seat 44b and the driving member 45b in the third embodiment, as follows:

In the fourth embodiment, the fixing seat 44c includes the first side wall 4410 facing the driving member 45c, and the plurality of elastic elements 46a are arranged around the connecting aperture 442 at the first side wall 4410, and the plurality of elastic elements 46a are arranged in a circle along the circumferential direction of the connecting aperture 442. Preferably, the plurality of elastic elements 46a are arranged at uniform intervals. Each elastic element 46a is an inclined convex rib, the convex rib is flexible, and can be elastically deform when squeezed. Each elastic element 46a includes the connecting block 467 protruding from the first side wall 4410 and the elastic positioning strip 468 obliquely extending from an end of the connecting block 467 to one side. The inclination angle of the elastic positioning strip 468 relative to the corresponding connection block 467 is within a range of 0° to 90° ; preferably, the inclination angle of the connection block 467 corresponding to the elastic positioning strip 468 is about 30° . The angle indexing part 47b is a toothed ring disposed between the driving cylinder 453 and the connecting cylinder 451, and the toothed ring surrounds the connecting cylinder 451 in a circle. The toothed ring includes a plurality of toothed grooves as the indexing elements 471b, and the plurality of toothed grooves can be matched with the convex ribs. Preferably, the plurality of toothed grooves are arranged in a circle at uniform intervals along the circumferential direction of the toothed ring. The method of application and beneficial effects of the fourth embodiment are the same as those of the third embodiment, and will not be repeated here.

In other embodiments, the plurality of indexing elements can be the plurality of grooves arranged at intervals at the second side wall 4531 of the driving part 453a, and the plurality of grooves are matched with the plurality of convex ribs arranged at the first side wall 4410 of the connecting ring 441.

### Fifth embodiment

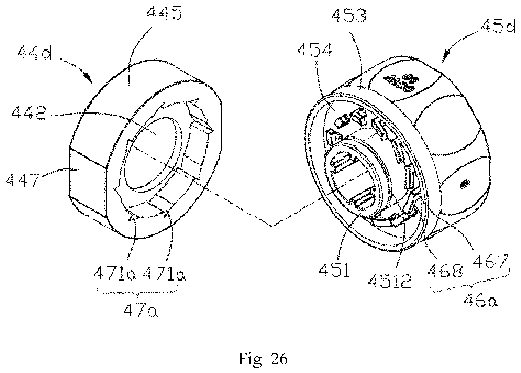

Please refer to Fig. 26, the structure of the fifth embodiment of the bendable sheath provided in the present application is similar to that of the fourth embodiment, the difference is that: the structure of the fixing seat 44d and the driving member 45d in the fifth embodiment is slightly different from the structures of the fixing seat 44c and the driving member 45c in the fourth embodiment, as follows:

In the fifth embodiment, the side of the fixing ring 445 of the fixing seat 44d facing the driving member 45d is provided with a plurality of indexing elements 471a at the peripheral side of the connecting aperture 442, and the plurality of indexing elements 471a are the plurality of grooves arranged at intervals. The plurality of grooves may be arranged at uniform intervals, or may be arranged at non-uniform intervals. The plurality of elastic elements 46a are arranged at the connecting board between the end of the connecting cylinder 451 of the driving member 45d and the driving cylinder 453, and the plurality of elastic elements 46a are arranged in a circle along the circumferential direction of the connecting cylinder 451, and each elastic element 46a is the flexible convex ribs; the plurality of convex ribs match the plurality of grooves.

It should be noted that, on the premise of not departing from the principles of the embodiments of the present application, the specific technical solutions in the above embodiments can be applied to each other, and will not be repeated here.

The above is the implementation of the embodiment of the present application, it should be pointed out that for those of ordinary skill in the art, without departing from the principle of the embodiment of the present application, some improvements and modifications can also be made, these improvements and modifications are also regarded as the scope of protection of the present application.

## Claims

1. A bendable sheath, comprising a tube assembly and a handle assembly, the handle assembly comprises an inner core, a fixing seat and a driving member, a distal end of the tube assembly is provided with a bendable section, a proximal end of the tube assembly is fixedly connected to the inner core, the driving member is fixedly connected to the inner core, the driving member is rotatably connected to the fixing seat, one of the fixing seat and the driving member comprises an elastic element, the other comprises an angle indexing part, the angle indexing part comprises a plurality of indexing elements arranged at intervals, when the driving member rotates relative to the fixing seat, the elastic element moves among the plurality of indexing elements.

2. The bendable sheath according to claim 1, wherein the plurality of indexing elements are arranged at equal intervals or unequal intervals.

3. The bendable sheath according to claim 1, wherein the fixing seat comprises a connecting ring, the connecting ring comprises a connecting aperture penetrating through the fixing seat in the axial direction, the driving member comprises a connecting cylinder, the connecting cylinder is rotatably disposed in the connecting aperture.

4. The bendable sheath according to claim 3, wherein the driving member comprises a driving cylinder coaxial with the connecting cylinder, an annular groove is formed between the driving cylinder and the connecting cylinder, the connecting ring is rotatably accommodated in the annular groove, the elastic element is disposed at the outer peripheral wall of the connecting ring, the plurality of indexing elements are disposed at the inner peripheral wall of the driving cylinder; or
the elastic element is disposed at the inner peripheral wall of the driving cylinder, the plurality of indexing elements are disposed at the outer peripheral wall of the connecting ring.

5. The bendable sheath according to claim 4, wherein the elastic element is selected from a spring plunger or an elastic protrusion, and the plurality of indexing elements are a plurality of concave grooves arranged at intervals, when the driving member rotates relative to the fixing seat, the spring plunger or the elastic protrusion is sequentially snapped into the plurality of concave grooves.

6. The bendable sheath according to claim 5, wherein the outer peripheral wall of the connecting ring or the inner peripheral wall of the driving cylinder is provided with an installation aperture along the radial direction, the elastic element is the spring plunger disposed in the installation aperture.

7. The bendable sheath according to claim 4, wherein the elastic element is a convex rib, the plurality of indexing elements are selected from a plurality of grooves or toothed grooves arranged at intervals, when the driving member rotates relative to the fixing seat, the convex rib is sequentially snapped into the plurality of grooves or the plurality of toothed grooves.

8. The bendable sheath according to claim 3, wherein the connecting ring comprises a first side wall facing the driving member, the driving member comprises a driving part connected to the connecting cylinder, the driving part is provided with a second side wall facing the first side wall around the connecting cylinder, the elastic element is disposed at the first side wall, the plurality of indexing elements are disposed at the second side wall; or,
the elastic element is disposed at the second side wall, the plurality of indexing elements are disposed at the first side wall.

9. The bendable sheath according to claim 8, wherein the elastic element is selected from a spring plunger or an elastic protrusion, the plurality of indexing elements are a plurality of concave grooves arranged at intervals, when the driving member rotates relative to the fixing seat, the spring plunger or the elastic protrusion is sequentially snapped into the plurality of concave grooves.

10. The bendable sheath according to claim 9, wherein the first side wall or the second side wall is provided with an installation aperture in the axial direction, the elastic element is the spring plunger disposed in the installation aperture.

11. The bendable sheath according to claim 8, wherein the elastic element is a convex rib, the plurality of indexing elements are selected from a plurality of grooves or toothed grooves arranged at intervals, when the driving member rotates relative to the fixing seat, the convex rib is sequentially snapped into the plurality of grooves or the plurality of toothed grooves.

12. The bendable sheath according to claim 3, wherein the handle assembly further comprises a circlip, the outer peripheral wall of the connecting cylinder is provided with a snapping groove, the connecting cylinder passes through the connecting aperture and exposes the snapping groove, the circlip is snapped into the snapping groove.

13. The bendable sheath according to claim 3, wherein a proximal end of the inner core is passed through and fixedly connected to the connecting cylinder.

14. The bendable sheath according to claim 13, wherein the handle assembly further comprises a fixing member, the proximal end of the inner core is provided with a plurality of locking pieces, the fixing member is provided with a locking interface, the proximal end of the inner core passes through the connecting cylinder and exposes the plurality of locking pieces, the plurality of locking pieces are inserted into the locking interface.

15. The bendable sheath according to claim 13, wherein the proximal end of the inner core is further provided with a plurality of locking slots, the inner peripheral wall of the connecting cylinder is provided with a plurality of locking ribs, the plurality of locking ribs and the plurality of locking slots are corresponding to each other and connected with each other.
